# EUROPÄISCHE PATENTANMELDUNG

(11) **EP 1 772 125 A1**
(43) Veröffentlichungstag der Anmeldung: **11.04.2007**
(21) Anmeldenummer: 06019300.0
(22) Anmeldetag: 15.09.2006
(51) Int. Cl.: A61F 11/08

(54) **Bügelgehörschutz**

(30) Priorität: 05.10.2005 DE 102005047589
(71) Anmelder: Uvex Arbeitsschutz GmbH, 90766 Fürth (DE)
(72) Erfinder: Brück, Stefan, 90419 Nürnberg (DE)
(74) Vertreter: Schneck, Herbert

(57) **Zusammenfassung**

Bei einem Bügelgehörschutz umfassend einen eigenelastischen, im Wesentlichen U-förmigen Kunststoffbügel mit endseitig angeordneten Ohrstöpseln mit einem Schaumstoffstöpsel, der im getragenen Zustand den Eingang des Gehörkanals verschließt, ist zur Verbesserung des Tragekomforts und der Gehörschutzeigenschaften vorgesehen, dass die Ohrstöpsel (4) bzw. Schaumstoffstöpsel (7) der Ohrstöpsel (4) in der Aufsicht eine oval-elliptische Grundform aufweisen.

## Beschreibung

Die Erfindung richtet sich auf einen Bügelgehörschutz umfassend einen eigenelastischen, im Wesentlichen U-förmigen Kunststoffbügel mit endseitig angeordneten Ohrstöpseln mit einem Schaumstoffstöpsel, der im getragenen Zustand den Eingang des Gehörkanals verschließt. Derartige Bügelgehörschutz-Anordnungen sind beispielsweise bekannt aus DE 296 11 562 U1 und US-Des. 295 530.

Gehörschutzeinrichtungen der in Betracht stehenden Naht müssen kostengünstig herstellbar sein, einen wirksamen Gehörschutz gewährleisten und einen möglichst hohen Tragekomfort aufweisen.

Hiervon ausgehend liegt der Erfindung die Aufgabe zu Grunde, einen Bügelgehörschutz der in Betracht stehenden Art so zu verbessern, dass sowohl der Tragekomfort als auch die Gehörschutzeigenschaften verbessert werden.

Diese Aufgabe wird erfindungsgemäß dadurch gelöst, dass die Schaumstoffstöpsel in der Aufsicht eine oval-elliptische Grundform aufweisen.

Insbesondere ist vorgesehen, dass die Ohrstöpsel so an dem Bügel montiert sind, dass sich der Bügel im Tragezustand, wenn also die Schaumstoffstöpsel in das Ohr des Benutzers eingesetzt sind, parallel zur großen Längsachse der oval-elliptischen Schaumstoffstöpsel und damit bezogen auf den Kopf des Benutzers von hinten-oben nach vorne-unten erstrecken.

Durch die oval-elliptische Formgebung wird zum einen eine optimale Anpassung an die Anatomie des Gehörgang-Eingangs erreicht, die deutlich besser ist als bei herkömmlichen in der Aufsicht runden Schaumstoffstöpsel, und zum anderen eine automatische Positionierung des Bügels derart, dass sich dieser um das Kinn des Benutzers herum erstreckt, so dass er einerseits die Augen bzw. das Sichtfeld und den Mund des Benutzers nicht stört, zum anderen auch nicht auf der Brust des Benutzers aufliegt, wodurch ein Schaden und die Weiterleitung von Schall verhindert wird.

In weiterer Ausgestaltung der Erfindung ist vorgesehen, dass die Schaumstoffstöpsel auf einen Trägerelement sitzen.

Das Trägerelement kann mittels einer Ringwulst in eine korrespondierende Nut an den Enden des Bügels aufgerastet werden, wobei natürlich auch eine kinematische Vertauschung von Wulst und Nut möglich ist.

Der Schaumstoffstöpsel seinerseits weist eine Ausnehmung auf, in die ein Vorsprung des Trägerelements formschlüssig eingreift, so dass der Schaumstoffstöpsel auch als solcher leicht auswechselbar ist.

Auf Grund eines Ringkragens, dessen Außenfläche mit einem konischen Abschnitt des Schaumstoffstöpsels fluchtet, ist eine sichere und saubere Befestigung gewährleistet.

Der Schaumstoffstöpsel besteht vorzugsweise aus Polyurethan.

Nachfolgend wird die Erfindung anhand eines bevorzugten Ausführungsbeispiels in Verbindung mit der Zeichnung näher erläutert. Dabei zeigen:

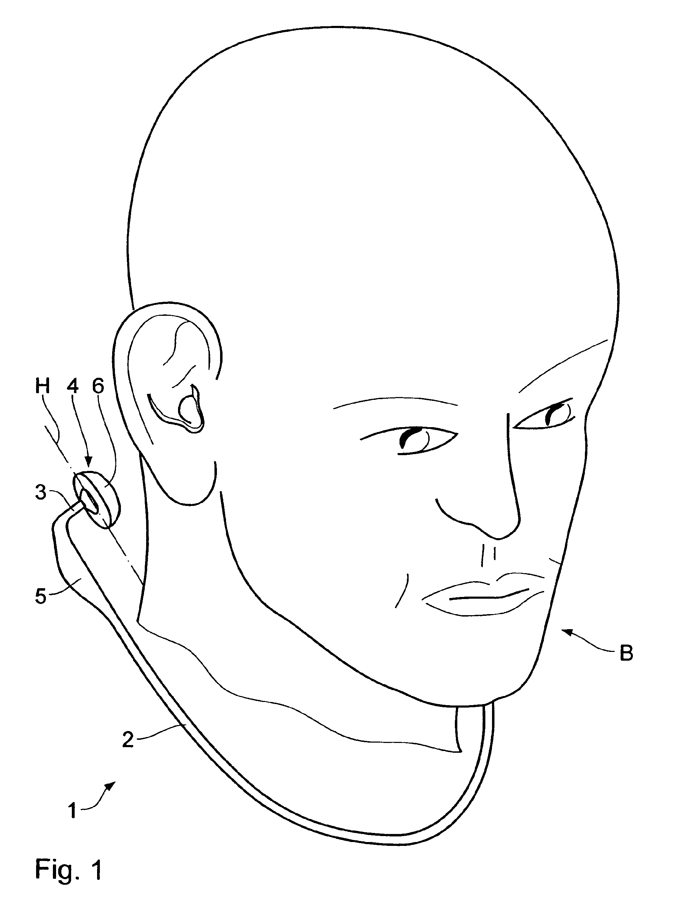

- Fig. 1: eine perspektivische Ansicht eines erfindungsgemäßen Bügelgehörschutzes in einer Position vor dem Aufsetzen,

- Fig. 2: eine Ansicht im aufgesetzten Zustand und
- Fig. 3: einen Schnitt durch den Ohrstöpsel senkrecht.

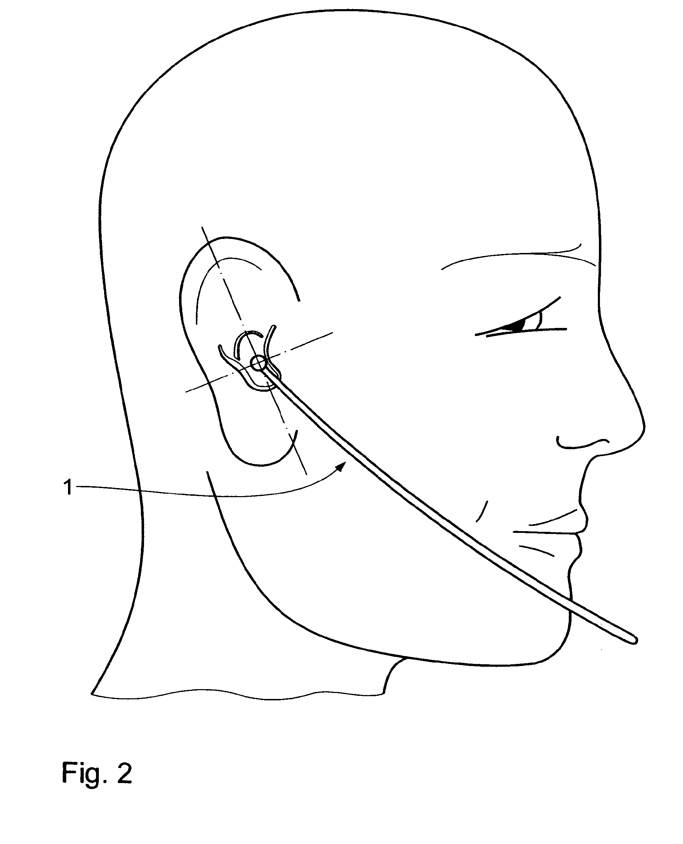

Ein in der Zeichnung dargestellter Bügelgehörschutz 1 umfasst einen einstückig aus Kunststoff gespritzten Bügel 2 mit um annähernd 90° abgekröpften Enden 3, auf welche Ohrstöpsel 4 aufgesetzt sind. Der Bügel 2 weist im Bereich vor den abgekröpften Enden 3 jeweils eine Verbreiterung 5 auf, die das Ergreifen erleichtert und es ermöglicht, den elastischen Bügel 2 zum Aufsetzen auseinander zu ziehen, wobei nach dem Loslassen durch die eigenelastische Rückstellkraft des Bügels 2 die Ohrstöpsel 4 über ihre abgerundete Anlagefläche 6 gegen den Eingang des Ohrkanals des Benutzers B gedrückt werden. Durch die oval-elliptische Form der Ohrstöpsel 4 mit einer im eingesetzten Zustand schräg von hinten-oben nach vorne unten verlaufenden Hauptachse H und die federnde Anlage wird ein Rotieren des Bügelgehörschutzes 1 um den Ohrkanal vermieden, sodass es ausgeschlossen ist, dass der Bügel 2 durch Bewegung an der Kleidung scheuert und dadurch verursachte störende Geräusche in das Innere des Ohres übertragen werden. Es wird also eine stabile Trageposition erreicht, wie sie in Fig. 2 dargestellt ist.

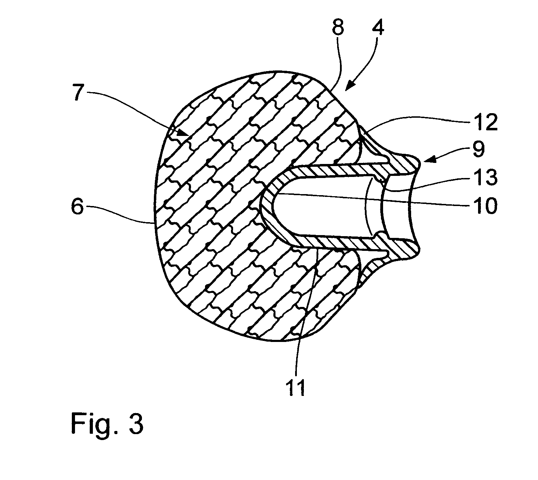

Wie insbesondere aus Fig. 3 zur erkennen ist, umfasst der Ohrstöpsel 4 einen ovalen Schaumstoffstöpsel 7 mit einer ovalen Anlagefläche 6, wobei der Schaumstoffstöpsel 7 nach außen hin einen konischen Endbereich 8 aufweist.

Ein Trägerelement 9, welches einstückig aus einem härteren Kunststoff gespritzt ist, greift mit einer Vorwölbung 10 in eine korrespondierende Ausnehmung 11 des Schaumstoffstöpsels 7 ein und besitzt einen Ringkragen 12, dessen Außenseite mit dem konischen Abschnitt 8 des Schaumstoffstöpsels 7 fluchtet.

An der Innenseite des Trägerelements 9 ist ein umlaufender Ringwulst 13 vorgesehen, der in eine korrespondierende, in der Zeichnung nicht dargestellte Ringnut an den abgekröpften Enden 3 des Bügels 2 einrastbar ist, sodass hierdurch eine auswechselbare Befestigung geschaffen wird.

## Patentansprüche

1. Bügelgehörschutz umfassend einen eigenelastischen, im Wesentlichen U-förmigen Kunststoffbügel mit endseitig angeordneten Ohrstöpseln mit einem Schaumstoffstöpsel, der im getragenen Zustand den Eingang des Gehörkanals verschließt, **dadurch gekennzeichnet, dass** die Ohrstöpsel (4) bzw. Schaumstoffstöpsel (7) der Ohrstöpsel (4) in der Aufsicht eine oval-elliptische Grundform aufweisen.

2. Bügelgehörschutz nach Anspruch 1, **dadurch gekennzeichnet, dass** die Ohrstöpsel (4) so an dem Bügel (2) montiert sind, dass sich der Bügel (2) im Tragezustand, wenn die Schaumstoffstöpsel (7) in das Ohr des Benutzers eingesetzt sind, parallel zur großen Längsachse des oval-elliptischen Schaumstoffstöpsels (7) und damit bezogen auf den Kopf des Benutzers B von hinten-oben nach vorne-unten erstrecken.

3. Bügelgehörschutz nach Anspruch **1, dadurch gekennzeichnet, dass** die Schaumstoffstöpsel (7) auf einem Trägerelement (9) sitzen.

4. Bügelgehörschutz nach Anspruch 3, **dadurch gekennzeichnet, dass** das Trägerelement (9) mittels einer Ringwulst (13) in eine korrespondierende Nut an den Enden (3) des Bügels (2) einrastbar ist.

5. Bügelgehörschutz nach Anspruch 3, **dadurch gekennzeichnet, dass** das Trägerelement (9) einen Ringkragen (12) aufweist, der mit einem konischen Abschnitt (8) des Schaumstoffstöpsels (7) fluchtet.

6. Bügelgehörschutz nach Anspruch 1, **dadurch gekennzeichnet, dass** die Schaumstoffstöpsel (7) aus Polyurethan bestehen.
